# EUROPEAN PATENT SPECIFICATION

(11) **EP 3 806 953 B1**
(45) Date of publication and mention of the grant of the patent: **30.10.2024**
(21) Application number: 19819374.0
(22) Date of filing: 14.06.2019
(51) Int. Cl.: A61N 5/06, G02C 11/04, G02C 5/00

(54) **A DEVICE FOR VISUAL STIMULATION**
VORRICHTUNG ZUR VISUELLEN STIMULATION
DISPOSITIF DE STIMULATION VISUELLE

(30) Priority: 15.06.2018 FI 20185543
(43) Date of publication of application: 21.04.2021
(73) Proprietor: Aalto University Foundation sr, 00076 Aalto (FI)
(72) Inventor: JOUSMÄKI, Veikko, 00076 Aalto (FI)
(74) Representative: Berggren Oy
(86) International application number: PCT/FI2019/050462
(87) International publication number: WO 2019/239015

(56) References cited:
- JP-A- 2007 185 326
- JP-A- 2007 185 326
- US-A- 5 414 459
- US-A1- 2005 010 266
- US-A1- 2010 121 158
- US-A1- 2013 182 085
- US-A1- 2015 018 927
- US-A1- 2015 297 444
- US-A1- 2016 106 950
- US-A1- 2016 106 950
- US-A1- 2018 133 504

## Description

### TECHNICAL FIELD OF THE INVENTION

The present invention relates to visual stimulation devices.

### BACKGROUND OF THE INVENTION

Intermittent photic stimulations (IPS) is a clinical standard in electroencephalographic (EEG) studies. Typically, IPS is produced with a stroboscope, display or led goggles with IPS frequencies from 1-50 Hz. During the clinical test the effect of IPS is evaluated both in eyes are can be open (EO) and eyes closed (EC) conditions. IPS is evaluated by observing cortical EEG signals and how they are driven by the IPS as a function of stimulus frequency. IPS is considered to enhance the diagnostic sensitivity of the EEG and be reasonably safe. In some cases, i.e. photosensitive epilepsy, it may result in epileptic seizures.

IPS has not been widely used in magnetoencephalographic (MEG) functional magnetic resonance imaging (fMRI), and magnetic resonance spectroscopy (MRS) studies since the commercially available IPS stimulators are not compatible with these neuroimaging methods and thus clinical standards are not available.

The existing solutions for IPS also occupy or obstruct the field of vision and thus do not allow a combination of foveal visual stimulation combined with simultaneous diffuse IPS in the peripheral vision.

Typically, IPS is generated by using stroboscopes, flash lights, cathode ray tube (CRT) displays, thin-film transistor (TFT) liquid-crystal displays, light-emitting diodes (LEDs), TFT projectors, or digital-light processing (DLP) projectors. IPS can be delivered through the lens of the eye in eyes open condition, i.e., direct foveal and Ganzfeld stimulation, or through the eyelid, i.e., diffuse peripheral stimulation in eyes closed condition. IPS elicits retinal (electroretinography, ERG) and cortical responses (visually evoked potentials (VEPs) in EEG, visually evoked fields (VEFs) in MEG). Typically, these standard IPS techniques are associated with electromagnetic interferences and may cause artefacts in sensitive functional neuroimaging modalities and thus they are not directly compatible with the functional neuroimaging modalities, e,g, MEG, fMRI, and MRS.

These standard IPS techniques do not allow foveal visual stimulation combined with simultaneous diffuse IPS stimulation in the peripheral vision in eyes open condition. In addition, they do not allow similar diffuse stimulation through the eyelid both in eyes closed and eyes open conditions. Furthermore, concomitant or interleaved visual stimulation without obstructing the field of view is not feasible in standard IPS experiments.

IPS is a gold standard in clinical electroencephalographic (EEG) protocols (Sinha et al., 2016; Martins da Silva et al., 2017) whereas it is not included in clinical magnetoencephalographic (MEG) protocols (Bagic et al., 2011; Burgess et al., 2011).

Document JP 2007 185326 A discloses spectacles enabling electrophysiological monitoring of visual function in neurosurgery under anesthesia. The spectacles are equipped with light sources providing visual stimulation to the retina the wearer. The spectacles are MRI- and CT- compatible.

Typically, the analysis of the IPS in EEG is limited to the non-statistical analysis describing localized and lateralized IPS driven activity in the occipital regions of the brain, generalized IPS driven activity in the brain, and periocular myoclonic activity.

### SUMMARY OF THE INVENTION

An object of the invention is to alleviate at least some of the problems of the prior art. In accordance with one aspect of the present invention, there is provided a device for visual stimulation, wherein the device comprises at least one light source and at least one light guide to direct light emitted by the at least one light source to at least one light outlet. The device comprises attachment means with which the light outlet is supportable and/or attachable in connection with a skin around an eye of a subject and the device is configured to direct, via the at least one light outlet, light emitted by the at least one light source, towards optically sensitive parts of the eye through the skin and/or tissue around the eye when the attachment means, the light outlet and/or light guide is in use position in connection with the skin around the eye.

The solution of the invention can be used e.g. for non-invasive functional neuroimaging in basic and clinical research, psychophysical experiments, neuromodulation, and diagnostics. In particular, the invention relates to devices for delivering retinal photic stimulation, constant or intermittent. The fields of application are in psychophysical and functional neuroimaging studies using, e.g., electroencephalography (EEG), magnetoencephalography (MEG), functional magnetic resonance imaging (fMRI), magnetic resonance spectroscopy (MRS), and navigated transcranial magnetic stimulation (nTMS).

In an embodiment, the invention may provide a stimulation device to provide diffuse retinal photic stimulation through the eyelids. The device has means to direct light towards the eyelids both in eyes open and eyes closed conditions. In eyes open condition, the stimulation may be given through the eyelid without obstructing the field of view, or only slightly obstructing the field of view.

The invention can use any light source. Especially in sensitive functional neuroimaging settings the light source can be located at a distance, and IPS can be delivered using a light guide such as a multifilament optic fiber. All the components of the device in the neuroimaging area can be made of materials that do not interfere with the imaging. Furthermore, no electrical currents are present in the parts of the device located within the neuroimaging area, which could be a source of interference.

Many benefits can be achieved by embodiments of the invention.

The stimulation device can present IPS diffusively, through the eyelid, facilitating similar IPS in the peripheral vision both in eyes open and eyes closed conditions without obstructing the field of view allowing concomitant and interleaved foveal visual stimulation and diffuse peripheral IPS.

The ability to use the stimulation devices in magnetic neuroimaging settings will facilitate international standards for IPS in MEG, fMRI, MRS, and nTMS. In addition, the database to be collected with the IPS stimulator embodiments will allow statistical comparison to the data obtained from patients and control subjects in EEG, MEG, fMRI, MRS, and nTMS. Cortical excitation index based on the IPS stimulation in functional neuroimaging measurements can also be evaluated quantitatively, respectively.

Furthermore, embodiments of the invention make it feasible to provide wearable IPS devices for neuromodulation, rehabilitation, and treatment using diffuse IPS, constant or intermittent, through the eyelid. The fields of applications are, e.g., in basic and clinical functional neuroimaging and psychophysical research in healthy population and in neurodegenerative diseases, e.g., in Alzheimer's and Parkinsonian diseases.

Cortical activity associated with the use of embodiments of the invention may be localized noninvasively in the brain using source modelling tools of a given functional neuroimaging tool and structural information obtained with magnetic resonance imaging. The resulting data can be used to quantify cortical excitability noninvasively in EEG, MEG, fMRI, MRS, and nTMS studies.

Multifiber optic fiber is a preferred way of directing light from the light source towards the subject. Any suitable light guides can be used for the purpose as is apparent to those skilled in the art.

Eyelids refer to the upper and lower eyelids, and more generally to the area of skin around the eyes from where light can pass through the tissue towards optically sensitive parts of the eye. Examples include the area of skin between the eye and the eyebrow, or the area of skin close to the eye, within the area of the orbit or eye socket of the human skull. Embodiments of the stimulation device can have light outlets on or in close proximity to any of these areas.

Light can be projected towards the subject from light outlets that can be the ends of the optic fibers, or also formed by additional components affixed to the ends of the optic fibers.

The exemplary embodiments presented in this text are not to be interpreted to pose limitations to the applicability of the appended claims. The verb "to comprise" is used in this text as an open limitation that does not exclude the existence of unrecited features.

The novel features which are considered as characteristic of the invention are set forth in particular in the appended claims. The invention itself, however, both as to its construction and its method of operation, together with additional objects and advantages thereof, will be best understood from the following description of specific example embodiments when read in connection with the accompanying drawings.

### BRIEF DESCRIPTION OF THE DRAWINGS

Figure 1 shows an exemplary embodiment of a stimulation device with the subject,
Figure 2 shows the placing and direction of the optical fiber relative to the eye according to an exemplary embodiment of the invention,
Figure 3 shows a supporting frame according to an exemplary embodiment with the ability to accommodate three optical fibers for each eye,
Figure 4 shows a multifilament optic fiber connected to a LED light source according to an exemplary embodiment of the invention,
Figure 5 shows multifilament optic fibers connected to a supporting frame eyepiece for holding the ends of the multifilament optic fibers with the light outlets close to the eyelid according to an exemplary embodiment of the invention,
Figure 6 shows a supporting frame according to an exemplary embodiment of an invention, with holes for holding multifilament optic fibers,
Figure 7 shows an exemplary embodiment of a stimulation device with the subject, and
Figure 8 shows the placing of the attachment means relative to the eye according to an exemplary embodiment of the invention.

### DETAILED DESCRIPTION

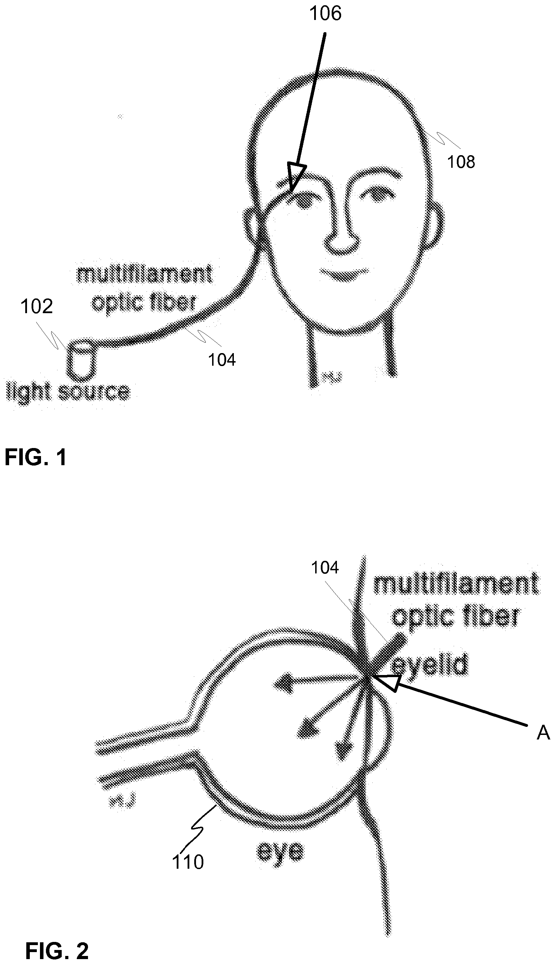

Figure 1 shows an embodiment of a device for visual stimulation, wherein the device comprises at least one light source 102, such as a light emitting diode, and at least one light guide 104. The light guide 104 is configured to direct light emitted by the at least one light source 102 to at least one light outlet 106.

In one embodiment of the invention the light source forms Intermittent photic stimulations (IPS). In one embodiment of the invention the stimulation can be constant or intermittent light

The device comprises support means or attachment means with which the light outlet 106 is supportable in connection with an eye of a subject 108. When the light outlet 106, light guide 104, and/or attachment means is arranged to be supported in connection with the skin around the eye, the device is in its use position.

In one embodiment of the invention support means may comprise attachment means for attaching the support means and/or the light guide 104 and/or light outlet 106 to the skin and/or tissue of the subject 108.

In one embodiment of the invention, in its use position (i.e. when the light outlet light guide, and/or attachment means is arranged to be supported in connection with the eye or the skin around the eye), the device is configured to direct, via the at least one light outlet 106, light emitted by the at least one light source 102 towards the eye of the subject 108 through the skin and/or tissue around the eye, e.g. through eyelid. The light guide 104 may be an optical fiber. The light guide may be a multifiber optical fiber.

The light outlet 106 may be formed by the side or end of the light guide 104. In one other embodiment, the light outlet may be formed by an additional component coupled to the light guide.

Yet, figure 1 shows an embodiment of the stimulation device in use position with a subject. The device comprises a light source, and a light guide such as a multifilament optic fiber. The device may also comprise attachment means (not shown) to attach the optic fiber on or in proximity of the eyelid of the subject. Attachment means may comprise adhesive materials such as tape, glue attaching the device to the user. Attachment means or support means may alternatively or in addition comprise a supporting frame as will be described in more detail later.

In a preferred embodiment there may be two optic fibers, one to provide stimulation to each eye. In this case the optic fibers can be attached to the same light source. Two optic fibers can also be split from one optic fiber using an optical component.

That is to say, in one embodiment of the invention light guide can be e.g. multifilament optical fiber. In one embodiment of the invention the device can comprise at least two optic fibers and one fiber can provide stimulation to each eye. When multiple light guides, e.g. optical fibers, are used they can be connected to same light source. In one embodiment of the invention two optic fibers can also be split from one optic fiber using an optical component. In one embodiment, when multiple light guides, e.g. optical fibers, are used, they can be connected to separate light sources.In one embodiment of the invention the light outlets are arranged to the ends of the light guide, e.g. optic fiber, and/or at the side of the light guide. Light outlet can be integral part of the light guide or it can be a separate part from the light guide. In one embodiment of the invention separate components can be used to direct light from the light guide to be projected towards the person, comprising light outlets and e.g. optical and mechanical components. It's also possible to use multiple light guides, e.g. optic fibers, and/or light outlets for each eye, e.g. 2 - 10 or e.g. 3-5.

The multifilament optic fibers can be placed on the eyelid for diffuse peripheral stimulation of the retina both in the eyes closed and eyes open conditions. With a preferred embodiment of the invention, the user may have eyes open or close them while the device is attached. Thus the device can provide stimulation both in eyes closed and eyes open conditions.

The optic fiber can also be attached on or near other areas around the eye such as the lower eyelid. There can also be several fibers used. For example, in an embodiment three fibers can be used for each eye.

In one embodiment of the invention the device further comprises means for other visual stimulation, e.g. foveal visual stimulation, which can be provided simultaneously with the stimulation directed to the eye via tissue around the eye and formed by the light source.

Figure 2 shows a side view of the eye 110 of the subject, and parts of an embodiment of the stimulation device. The end of an optic fiber 104 may be placed in a location that enables the light to travel through the tissue adjacent to and covering the eye 110. Little or no light is directed directly to the surface of the eye.

At Figure 2 (not showing e.g. the light source 102 or the attachment means), the end of the light guide 104 forms the light outlet 106 and the light outlet is arranged to be supported in connection with the eye 110 or in connection with the skin around the eye. Here, a location A is pointed out, which is a location of the light outlet, thereby giving an example of how the light outlet may be arranged to be supported in connection with the eye or in connection with the skin around the eye.

The location A in Figure 2 may be a point where the light outlet 106 is in contact or close proximity with the skin and/or tissue of the subject 108.

The light being directed towards the eye passes through the skin and/or tissue around the eye. Here, it may be understood that the location of the light outlet when the device is in its use position is such that the light may pass through the skin when the subject has their eyes closed and when the subject has their eyes open.

The light outlet 106, when arranged to be supported in connection with the eye or in connection with the skin around the eye, is preferably located so that it essentially does not obstruct a visual field of the subject 108.

In one embodiment of the invention light outlet is arranged on the area of skin around the eyes so that light can pass through the tissue towards optically sensitive parts of the eye. Light outlet can be arranged so that essentially no light is directed directly to the surface of the eye when the device is positioned on the head of the person, i.e., when in its use position. In one embodiment of the invention, when the device is positioned on the person, light outlet is arranged so that it is located on the eyelid, e.g. in the area between the eye and the eyebrow. Light outlet can be in direct contact with the skin but it's also enough that the light outlet in the proximity of the skin so that the light can enter the eye via the skin and/or tissue. In one embodiment of the invention the light outlet is slightly above the eyelid, such as 0 - 50 mm, 0-10 mm, or 0 - 5 mm above the eyelid. The light outlet may form a single entry point or multiple entry points through the eyelid or skin around the eye.

In one embodiment of the device, the supporting means may comprise a frame element or supporting frame that may constitute a wearable device.

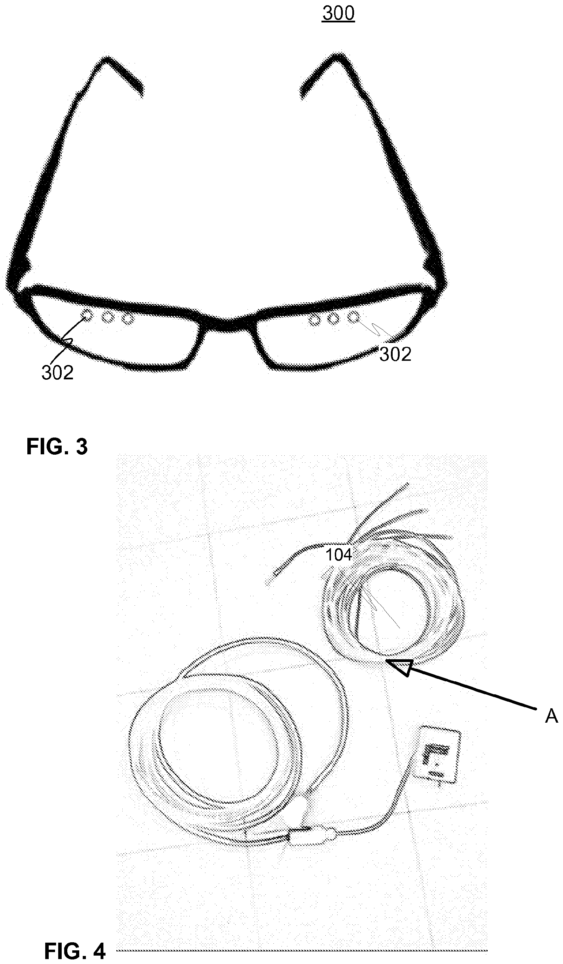

Figure 3 shows an exemplary embodiment of a supporting frame and/or supporting element 300 for attaching the stimulation device to the user or for supporting the device in connection with the eye or in connection with the skin around the eye of a subject.

The supporting frame may comprise attachment means for attaching the light outlet(s) to or near the eye(s) of the subject or the supporting means may comprise other elements for arranging the light outlet to be supported in connection with the eye or in connection with the skin around the eye.

A supporting frame for the purpose can be designed to be held in place by physical features of the subject, such as one or several of the head, ears, nose, cheeks. The desired number of optic fibers can be attached to the supporting frame.

A supporting frame 300 may comprise light outlet coupling points 302 where the light guides 104 and/or light outlets 106 may be coupled to the supporting frame 300.

Figure 4 shows a multifilament optic fiber connected to a LED light source according to an exemplary embodiment of the invention.

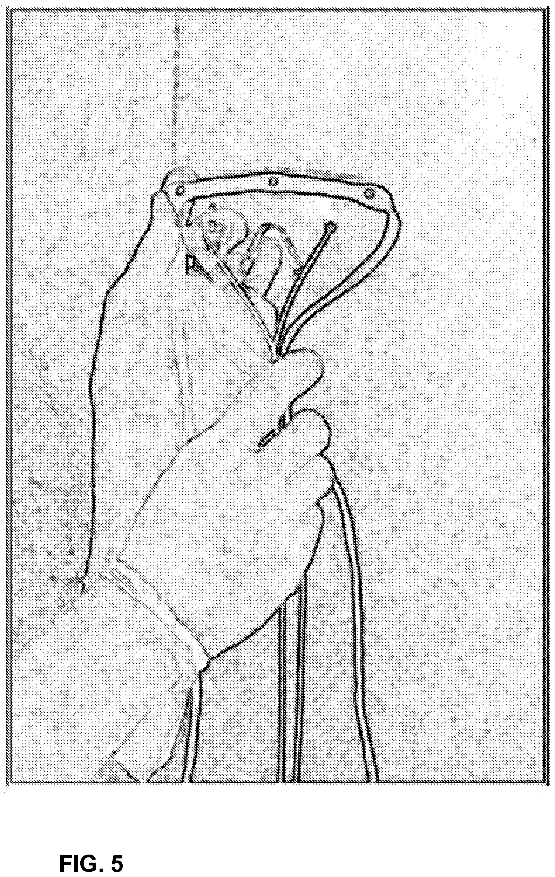

Figure 5 shows multifilament optic fibers connected to a supporting frame eyepiece for holding the ends of the multifilament optic fibers with the light outlets close to the eyelid according to an exemplary embodiment of the invention.

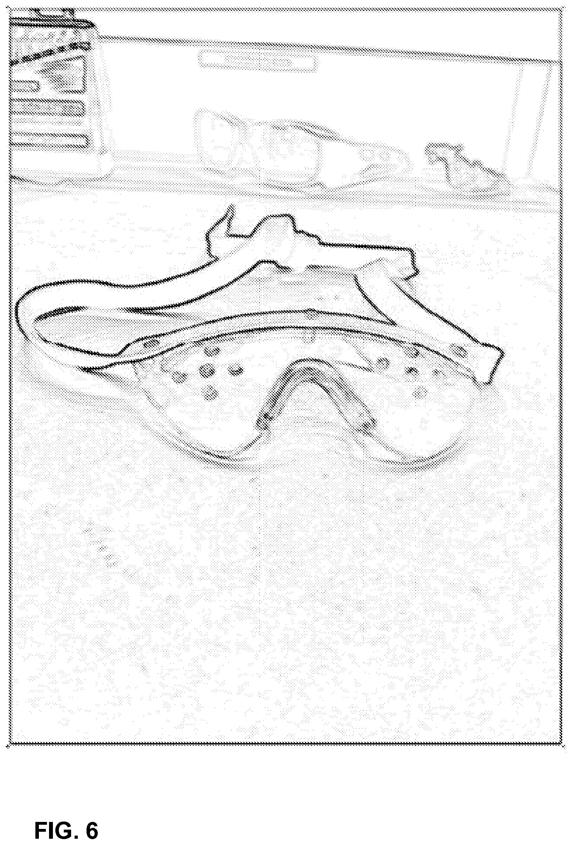

Figure 6 shows a supporting frame according to an exemplary embodiment of an invention, with holes for holding multifilament optic fibers.

A part of a wearable device attached to the subject or arranged to be supported in connection with the eye or in connection with the skin around the eye of a subject can be lightweight. This allows for comfortable wearing. This can be beneficial especially when long stimulation sequences are used and allowing the subject to comfortably view other visual stimuli in a test setting, or also perform other tasks while receiving therapeutic IPS. In a preferred embodiment, the device is constructed so that the subject may move their head relatively freely while the stimulator is in place. The required range of motion depends on the use case.

The device is small in size which makes it possible to use together with various types of neuroimaging systems which may occupy large volumes of the area in proximity of the head of the subject. The small size also allows for various therapeutic techniques to be applied while the stimulator is in place, without each interfering with the other.

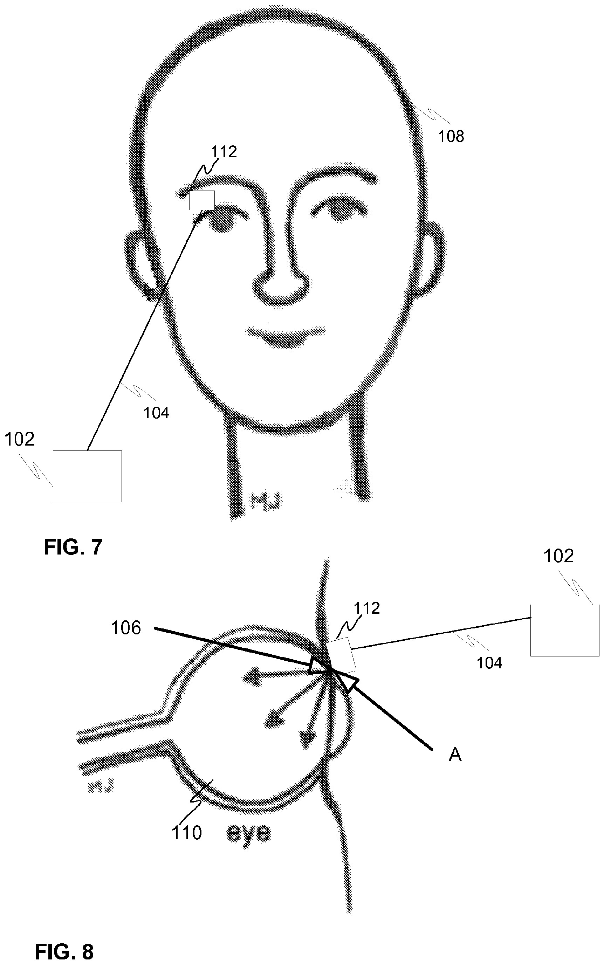

Figure 7 shows an exemplary embodiment of a stimulation device with the subject and Figure 8 shows the placing of the attachment means relative to the eye according to an exemplary embodiment of the invention. Figures 7 and 8 otherwise correspond Figures 1 and 2 but in these Figures 7 and attachment means 112 is presented which attaches the light guide and/or the light outlet in connection to the skin around the eye, e.g. eyelid, so that the light of the light source is directed via the light outlet to inside the eye through skin when the attachment means are in use position.

In example embodiments presented in Figures 7 and 8 a light outlet is supported and/or attached in connection with skin around the eye of a subject 108. Attachment means or support means 112 are used for attaching the light guide 104 and/or light outlet 106 in connection with the eye or in connection with the skin around the eye of a subject 108.

The attachment means used in the solution of the invention may also comprise or include one or several strings or bands. In a preferred embodiment the one or several strings or bands can be made of flexible material.

In one embodiment of the invention the attachment means can comprise a frame which helps in keeping the attachment means, the light outlet and/or light guide in use position in connection with the skin around the eye.

In the solution of the invention the attachment means can comprise at least one of the following: adhesive material, tape, frame, string, band. These attachment means can be combined such that the attachment means are capable to support and/or attach the light outlet in connection with the skin around the eye of the subject.

In one embodiment of the invention, light source is placed at minimum so far away from the light outlets that the light source and electronics connected to the light source doesn't cause interference to the imaging apparatus used in imaging.

In one embodiment of the invention the materials of light guide and light outlet are such that they don't cause interference for imaging. This way the device can be used in magnetoencephalography (MEG) environment.

Different types of light sources and light guides can be used for different embodiments of the invention.

For clinic use the light source can be accurate and set in a separate room to minimize electromagnetic interference.

For use for example in a home or healthcare setting, or in a portable setting where there are not high demands for low interference, the light source can be small and in closer proximity to the subject. In a portable embodiment, the light source can also be carried by the subject for example in a pocket or a purse or as a wearable device.

A benefit is that the same or very similar device can be used in clinical imaging settings for example to test a response to a treatment stimulus pattern, and then the same or very similar device can be used in a healthcare or home setting for administering recurring stimulus. Parts of the device can also be substituted, for example a different light source used in the clinical setting.

Embodiments of the stimulation device present novel possibilities for quantifying cortical excitability noninvasively in EEG, MEG, fMRI, and nTMS studies.

Another embodiment can be used for diffuse peripheral IPS through the eyelid in normal everyday activities for neuromodulatory purposes. In this embodiment the components and construction do not necessarily need to be compatible with functional neuroimaging methods.

Embodiments of the invention include devices for delivering retinal photic stimulation, constant or intermittent, using multifilament optical fibers for diffuse visual stimulation using small single entry point or multiple entry points through the eyelid both in eyes open and eyes closed condition without obstructing the visual field.

Quantification methods to evaluate the elicited photic response may include functional neuroimaging modalities, e.g, EEG, MEG, fMRI, MRS, and nTMS.

Embodiments of the device can be used in e.g. neuromodulation, rehabilitation, and treatment in healthy population and diseased, e.g., in neurodegenerative diseases.

Various benefits can be achieved by embodiments of the invention. The stimulation device may be used in functional neuroimaging environments as well as other environments sensitive to interference.

Another benefit of the invention is the possibility of foveal visual stimulation combined with simultaneous diffuse IPS stimulation in the peripheral vision.

Embodiments of the stimulation device enable novel possibilities to produce similar diffuse photic stimulation in the peripheral vision both in eyes open and eyes closed conditions.

Novel possibilities to use independent stochastic diffuse photic stimulation sequences to one or two eyes simultaneously or interleaved manner.

Embodiments of the invention do not obstruct vision and can be combined with visual stimulation.

Embodiments are potential tools for neuromodulation and quantifying cortical excitability.

Embodiments are compatible with functional neuroimaging methods.

Embodiments have the potential for gamma frequency entrainment to attenuate amyloid load and modify microglia.

An embodiment of the invention is a visual stimulation device for use in neuroimaging, wherein the stimulation device comprises means for transmitting light and directing it to the skin around the eye of the subject.

In an embodiment the stimulation device is at least partially constructed of materials that are suitable for magnetic imaging environments. In addition the device can be constructed in a way that no interference such as electric currents are present in the magnetic imaging environment.

Embodiments of the device may use a light guide such as an optic fiber to bring light from a light source to be projected to the subject through light outlets. The light outlets may be located on or in proximity of the skin of the subject.

The distance of the light guide or optic fiber may be relatively small for uses such as placing the light source to be carried by the user or placed on a table. In neuroimaging settings for example, the distance can be long to permit placing the light source in the area outside of the imaging area.

In an embodiment the device is used for intermittent photic stimulation during neuroimaging.

In a preferred embodiment the device can be fitted on the eyelid, in the area between the eye and the eyebrow as shown in Fig 2. The device may be arranged to be able to provide the stimulation in both eyes open and eyes closed condition. Preferably this can be achieved without moving the device. The device may be arranged such that when used in eyes open condition the device does not obstruct the visual field of the subject or obstructs it only minimally. The device may be attached by various means such as using adhesive materials, or a supporting frame. A supporting frame for the purpose can be designed to be held in place by physical features of the subject, such as the head, ears, nose, cheeks.

In an embodiment the device has light outlets from where the light is directed towards the subject. In an embodiment the light outlets are the ends of the optic fibers. In an embodiment the light outlets are the side of the optic fiber. In an embodiment, separate components are used to direct light from the light guide to be projected towards the user, comprising light outlets and optical and mechanical components.

In an embodiment, the light outlet is directly on the eyelid.

In another embodiment, the light outlet is slightly above the eyelid, such as 0-50mm, preferably 0-10mm, preferably 0-5mm.

In an embodiment, the optical fiber may be of a type that is open from the side. In this case the fiber would be attached to the similar position as otherwise but in a direction that facilitates the propagation of light towards the eyelid and from there towards the eye.

Endpieces for optical fibers can also be used and designed for changing the spreading of the light as well as the physical appearance and fit of the device for the subject.

In an embodiment the device comprises one optic fiber and one light outlet for each eye. The device may also comprise several optic fibers and/or light outlets for each eye, such as 2-10, 3-5. In this way the light can be distributed on a larger area.

Optic elements may be used to change the area of the light outlet or the direction of light to achieve the desired direction, intensity, beam size and characteristics of the light, for example to spread the light to a larger area. An embodiment of the invention is the use of one or more optical fibers for providing intermittent photic stimulation.

In an embodiment, the IPS stimulator provides diffuse photic stimulation, constant and intermittent, to the retina of the eye in eyes open and eyes closed conditions. The device is safe to use and compatible with psychophysical experiments and functional neuroimaging methods, e.g., EEG, MEG, fMRI, MRS, and nTMS. It can also be used to provide neuromodulatory stimulation.

The invention has been explained above with reference to the aforementioned embodiments, and several advantages of the invention have been demonstrated. The invention is defined in the following claims. Other items, embodiments, examples and methods are not a part of the invention.

## Claims

1. A device for visual stimulation, wherein the device comprises at least one light source (102) and at least one light guide (104) to direct light emitted by the at least one light source (102) to at least one light outlet (106), wherein the device comprises attachment means (112) with which the light outlet (106) is supportable and/or attachable in connection with a skin around an eye of a subject (108) and the device is configured to direct, via the at least one light outlet (106), light emitted by the at least one light source (102), towards optically sensitive parts of the eye through the skin and/or tissue around the eye so that the light outlet (106) is arranged to be located essentially outside of a visual field of the subject (108) when the attachment means (112), the light outlet (106) and/or light guide (104) is in use position in connection with the skin around the eye, wherein the light guide (104) and the light outlet (106) are made of materials that do not interfere with imaging in such a way that the device can be used in magnetic imaging environment, such as magnetoencephalography (MEG), functional magnetic resonance imaging (fMRI), and/or magnetic resonance spectroscopy (MRS) environment, and wherein the light source (102) is arranged at minimum so far away from the light outlets that the light source and electronics connected to the light source do not cause interference to an imaging apparatus used in imaging.

2. The device of claim 1, wherein the device comprises at least two light guides, wherein each light guide is configured to direct light emitted by the at least one light source to a respective light outlet, and wherein the attachment means is configured to support at least one light outlet in connection with skin around each eye of the subject.

3. The device of any previous claims, wherein the at least one light guide (104) is an optical fiber, optionally a multifilament optical fiber.

4. The device of any previous claims, wherein the light outlet (106) is formed by the side or end of the light guide or the light outlet is formed by an additional component coupled to the light guide.

5. The device of any previous claims, wherein the light source (102) is a light emitting diode.

6. The device of any previous claims, wherein the light source (102) is configured to emit essentially continuous light or flashes of light, e.g. Intermittent Photic Stimulation (IPS) light.

7. The device of any previous claims, wherein the attachment means (112) for attaching the light outlet and/or light guide to the skin and/or tissue of a subject comprises adhesive material.

8. The device of any previous claims, wherein the attachment means (112) comprises at least a frame element that constitutes a wearable device.

9. The device of any previous claims, wherein the attachment means (112) is configured to support at least the light outlet (106) so that the light outlet (106) is arranged to be located directly on the skin and/or tissue or at a distance from skin and/or the tissue, wherein the distance is between 0 and 50 mm, preferably between 0 and 10 mm, or more preferably between 0 and 5 mm, when the attachment means (112), the light outlet (106) and/or light guide (104) is in use position in connection with the skin around eye the.

10. The device of any previous claims, wherein the light outlet (106) is arranged so that essentially no light of the light source (102) is directed directly to the surface of the eye when the attachment means (112), the light outlet (106) and/or light guide (104) is in use position in connection with the skin around the eye.

11. The device of any previous claims, wherein the light outlet (106) is arranged so that the light of the light source (102) is directed via light outlet (106) to the eye through the skin and/or tissue around the eye both in eyes open and in eyes closed condition when the attachment means (112), the light outlet (106) and/or the light guide (104) is in use position in connection the skin around with the eye.

## Patentansprüche

1. Gerät zur visuellen Stimulation, wobei das Gerät mindestens eine Lichtquelle (102) und mindestens einen Lichtleiter (104) umfasst, um von der mindestens einen Lichtquelle (102) emittiertes Licht zu mindestens einem Lichtauslass (106) zu leiten, wobei das Gerät Befestigungsmittel (112) umfasst, mit dem der Lichtauslass (106) in Verbindung mit einer Haut um ein Auge eines Probanden (108) herum stützbar und/oder befestigbar ist, und das Gerät dazu konfiguriert ist, über den mindestens einen Lichtauslass (106) von der mindestens einen Lichtquelle (102) emittiertes Licht durch die Haut und/oder das Gewebe um das Auge herum auf optisch empfindliche Teile des Auges zu leiten, so dass der Lichtauslass (106) so angeordnet ist, dass er sich im Wesentlichen außerhalb eines Sichtfelds des Probanden (108) befindet, wenn sich das Befestigungsmittel (112), der Lichtauslass (106) und/oder der Lichtleiter (104) in Gebrauchsposition in Verbindung mit der Haut um das Auge herum befinden, wobei der Lichtleiter (104) und der Lichtauslass (106) aus Materialien bestehen, die die Bildgebung nicht beeinträchtigen, so dass das Gerät in einer Umgebung für magnetische Bildgebung, wie etwa einer Magnetoenzephalographie (MEG), einer funktionellen Magnetresonanztomographie (fMRI) und/oder einer Magnetresonanzspektroskopie (MRS), verwendet werden kann, und wobei die Lichtquelle (102) mindestens so weit von den Lichtauslässen entfernt angeordnet ist, dass die Lichtquelle und die mit der Lichtquelle verbundene Elektronik keine Störungen bei einem zur Bildgebung verwendeten Bildgebungsvorrichtung verursachen.

2. Gerät gemäß Anspruch 1, wobei das Gerät mindestens zwei Lichtleiter umfasst, wobei jeder Lichtleiter so konfiguriert ist, dass er von der mindestens einen Lichtquelle emittiertes Licht zu einem jeweiligen Lichtauslass leitet, und wobei das Befestigungsmittel so konfiguriert ist, dass es mindestens einen Lichtauslass in Verbindung mit der Haut um jedes Auge des Probanden hält.

3. Gerät gemäß einem der vorhergehenden Ansprüche, wobei der mindestens eine Lichtleiter (104) eine optische Faser, optional eine Multifilament-Lichtleitfaser, ist.

4. Gerät gemäß einem der vorhergehenden Ansprüche, wobei der Lichtauslass (106) durch die Seite oder das Ende des Lichtleiters gebildet wird oder der Lichtauslass durch eine zusätzliche Komponente gebildet wird, die mit dem Lichtleiter gekoppelt ist.

5. Gerät gemäß einem der vorhergehenden Ansprüche, wobei die Lichtquelle (102) eine Leuchtdiode ist.

6. Gerät gemäß einem der vorhergehenden Ansprüche, wobei die Lichtquelle (102) so konfiguriert ist, dass sie im Wesentlichen kontinuierliches Licht oder Lichtblitze, z. B. Intermittierendes fotostimulierendes Licht (IPS) aussendet.

7. Gerät gemäß einem der vorhergehenden Ansprüche, wobei die Befestigungsmittel (112) zum Befestigen des Lichtauslasses und/oder des Lichtleiters an der Haut und/oder dem Gewebe eines Subjekts Klebematerial umfassen.

8. Gerät gemäß einem der vorhergehenden Ansprüche, wobei die Befestigungsmittel (112) mindestens ein Rahmenelement umfassen, das ein tragbares Gerät darstellt.

9. Gerät gemäß einem der vorhergehenden Ansprüche, wobei das Befestigungsmittel (112) so konfiguriert ist, dass es zumindest den Lichtauslass (106) stützt, so dass der Lichtauslass (106) so angeordnet ist, dass er direkt auf der Haut und/oder dem Gewebe oder in einem Abstand von der Haut und/oder dem Gewebe angeordnet ist, wobei der Abstand zwischen 0 und 50 mm, vorzugsweise zwischen 0 und 10 mm oder noch besser zwischen 0 und 5 mm beträgt, wenn sich das Befestigungsmittel (112), der Lichtauslass (106) und/oder der Lichtleiter (104) in Gebrauchsposition in Verbindung mit der Haut um das Auge herum befinden.

10. Gerät gemäß einem der vorhergehenden Ansprüche, wobei der Lichtauslass (106) so angeordnet ist, dass im Wesentlichen kein Licht der Lichtquelle (102) direkt auf die Oberfläche des Auges gerichtet ist, wenn sich das Befestigungsmittel (112), der Lichtauslass (106) und/oder der Lichtleiter (104) in Gebrauchsposition in Verbindung mit der Haut um das Auge befinden.

11. Gerät gemäß einem der vorhergehenden Ansprüche, wobei der Lichtauslass (106) so angeordnet ist, dass das Licht der Lichtquelle (102) sowohl bei geöffneten als auch bei geschlossenen Augen über den Lichtauslass (106) durch die Haut und/oder das Gewebe um das Auge herum zum Auge geleitet wird, wenn sich das Befestigungsmittel (112), der Lichtauslass (106) und/oder der Lichtleiter (104) in Gebrauchsposition in Verbindung mit der Haut um das Auge herum befinden.

## Revendications

1. Dispositif de stimulation visuelle, dans lequel le dispositif comprend au moins une source de lumière (102) et au moins un guide de lumière (104) pour diriger une lumière émise par l'au moins une source de lumière (102) vers au moins une sortie de lumière (106), dans lequel le dispositif comprend un moyen de fixation (112) avec lequel la sortie de lumière (106) peut être supportée et/ou fixée en liaison avec une peau autour d'un œil d'un sujet (108) et le dispositif est configuré pour diriger, via l'au moins une sortie de lumière (106), une lumière émise par l'au moins une source de lumière (102), vers des parties optiquement sensibles de l'œil à travers la peau et/ou le tissu autour de l'œil de sorte que la sortie de lumière (106) est agencée pour être située essentiellement à l'extérieur d'un champ visuel du sujet (108) lorsque le moyen de fixation (112), la sortie de lumière (106) et/ou le guide de lumière (104) sont en position d'utilisation en liaison avec la peau autour de l'œil, dans lequel le guide de lumière (104) et la sortie de lumière (106) sont constitués de matériaux qui n'interfèrent pas avec l'imagerie de telle manière que le dispositif peut être utilisé dans un environnement d'imagerie magnétique, tel que la magnétoencéphalographie (MEG), l'imagerie par résonance magnétique fonctionnelle (IRMf) et/ou un environnement de spectroscopie par résonance magnétique (SRM), et dans lequel la source de lumière (102) est agencée au minimum si loin des sorties de lumière que la source de lumière et les composants électroniques reliés à la source de lumière ne provoquent pas d'interférence avec un appareil d'imagerie utilisé dans l'imagerie.

2. Dispositif selon la revendication 1, dans lequel le dispositif comprend au moins deux guides de lumière, dans lequel chaque guide de lumière est configuré pour diriger une lumière émise par l'au moins une source de lumière vers une sortie de lumière respective, et dans lequel le moyen de fixation est configuré pour supporter au moins une sortie de lumière en liaison avec la peau autour de chaque œil du sujet.

3. Dispositif selon l'une quelconque des revendications précédentes, dans lequel l'au moins un guide de lumière (104) est une fibre optique, éventuellement une fibre optique multifilament.

4. Dispositif selon l'une quelconque des revendications précédentes, dans lequel la sortie de lumière (106) est formée par le côté ou l'extrémité du guide de lumière ou la sortie de lumière est formée par un composant supplémentaire couplé au guide de lumière.

5. Dispositif selon l'une quelconque des revendications précédentes, dans lequel la source de lumière (102) est une diode électroluminescente.

6. Dispositif selon l'une quelconque des revendications précédentes, dans lequel la source de lumière (102) est configurée pour émettre une lumière essentiellement continue ou des flashs de lumière, par exemple une lumière de stimulation photique intermittente (IPS) .

7. Dispositif selon l'une quelconque des revendications précédentes, dans lequel le moyen de fixation (112) pour fixer la sortie de lumière et/ou le guide de lumière à la peau et/ou au tissu d'un sujet comprend un matériau adhésif.

8. Dispositif selon l'une quelconque des revendications précédentes, dans lequel le moyen de fixation (112) comprend au moins un élément de cadre qui constitue un dispositif portable.

9. Dispositif selon l'une quelconque des revendications précédentes, dans lequel le moyen de fixation (112) est configuré pour supporter au moins la sortie de lumière (106) de sorte que la sortie de lumière (106) est agencée pour être située directement sur la peau et/ou le tissu ou à une distance de la peau et/ou du tissu, dans lequel la distance est comprise entre 0 et 50 mm, de préférence entre 0 et 10 mm, ou de manière davantage préférée entre 0 et 5 mm, lorsque le moyen de fixation (112), la sortie de lumière (106) et/ou le guide de lumière (104) sont en position d'utilisation en liaison avec la peau autour de l'œil.

10. Dispositif selon l'une quelconque des revendications précédentes, dans lequel la sortie de lumière (106) est agencée de sorte qu'essentiellement aucune lumière de la source de lumière (102) n'est dirigée directement vers la surface de l'œil lorsque le moyen de fixation (112), la sortie de lumière (106) et/ou le guide de lumière (104) sont en position d'utilisation en liaison avec la peau autour de l'œil.

11. Dispositif selon l'une quelconque des revendications précédentes, dans lequel la sortie de lumière (106) est agencée de sorte que la lumière de la source de lumière (102) est dirigée via une sortie de lumière (106) vers l'œil à travers la peau et/ou le tissu autour de l'œil à la fois dans l'état yeux ouverts et dans l'état yeux fermés lorsque le moyen de fixation (112), la sortie de lumière (106) et/ou le guide de lumière (104) sont en position d'utilisation en liaison avec la peau autour de l'œil.
